# EUROPEAN PATENT APPLICATION

(11) **EP 2 937 330 A1**
(43) Date of publication of application: **28.10.2015**
(21) Application number: 15158387.9
(22) Date of filing: 03.11.2011
(51) Int. Cl.: C07C 51/265, C07C 51/44, C07C 51/48, C07C 63/15, C07B 63/00, B01D 3/40

(54) **Production of aromatic carboxylic acids**

(30) Priority: 22.11.2010 US 416071 P
(62) Divisional of application: 11842781.4
(71) Applicant: Invista Technologies S.à r.l., 9000 St. Gallen (CH)
(72) Inventor: Ure, Alan Macpherson, Cleveland, TS18 5DJ (GB); Tharakan, Ajay, Durham, DH1 5SE (GB)
(74) Representative: Carpmaels & Ransford LLP

(57) **Abstract**

Disclosed are processes and systems for the improved production of aromatic carboxylic acids, such as purified terephthalic acid. The processes result in the use of a smaller distillation device to more efficiently recover the carboxylic acid solvent throughout various stages of the PTA process when compared to known processes. The smaller distillation device is achieved by using downstream water treatment devices and organic compound extraction devices to further separate organic compounds from the aqueous byproduct streams.

## Description

### CROSS-REFERENCE TO RELATED APPLICATION

This application claims benefit of priority from U.S. Provisional Application No. 61/416,071 filed November 22, 2010.

### FIELD OF THE INVENTION

This invention relates to processes and systems for the production of aromatic carboxylic acids, such as purified terephthalic acid (PTA). One aspect of the invention concerns a more efficient method of producing aromatic carboxylic acids. Another aspect concerns a method to reduce the loss of process solvent in the production of aromatic carboxylic acids.

### BACKGROUND OF THE INVENTION

Aromatic polycarboxylic acids, such as terephthalic acid, are important chemical intermediates used for the production of industrially significant products, including polyester polymers, which can be used for fiber production and in the manufacture of containers, bottles and other molded articles.

Purified terephthalic acid (PTA) can be produced in a two stage process. Current technology for the manufacture of terephthalic acid involves the liquid phase oxidation of an aromatic feedstock, such as paraxylene, using molecular oxygen in a solvent. The oxidation solvent comprises a lower (e.g. C₂-C₆) aliphatic carboxylic acid, usually acetic acid and water, in the presence of a dissolved heavy metal catalyst system usually incorporating a promoter, such as bromine. Acetic acid is particularly useful as the solvent since it is relatively resistant to oxidation and increases the activity of the catalytic pathway for the oxidation of aromatic feedstock and reaction intermediates. The reaction is carried out in one or more stirred vessels under elevated temperature and pressure, in the range of about 150 to 250°C and 6 to 30 barA respectively and typically produces crude terephthalic acid (CTA) in high yield, e.g. at least 95%. Under these conditions the CTA precipitates from the solvent in the oxidation reactor to form a slurry of CTA solids in oxidation solvent, which is maintained in suspension by agitation in the reaction vessels. The temperature of the slurry is reduced by passing through a series of crystallizers, each at successively lower pressure, before the CTA solids are separated from the oxidation reaction solvent to give the oxidation mother liquor. The separation of the CTA solids from the oxidation mother liquor occurs at positive pressure or under vacuum.

Typically, the solvent for the liquid phase oxidation is aqueous acetic acid and contains water resulting from the oxidation of paraxylene and other reaction precursors. The oxidation reaction is exothermic and generates aromatic carboxylic acid, reaction intermediates from the partial oxidation of the aromatic feedstock, such as p-toluic acid (pTol) and by-products (comprising color-forming compounds), volatile components, such as methanol, methyl acetate and methyl bromide, and degradation products, such as carbon dioxide, carbon monoxide (carbon oxides) and benzoic acid (BA).

The second stage of the production process is the purification of CTA by catalytic hydrogenation in aqueous solution. Typically, CTA solids are dissolved in water at high pressure (70-90 barA) and high temperature (275 - 290° C), and hydrogenated over a fixed bed catalyst of palladium supported on carbon. The resulting solution is cooled as it passes through a series of crystallizers, where the purified terephthalic acid (PTA) is crystallized. The resulting slurry at a temperature in the range of about 140-160° C is fed to a suitable continuous solid liquid separation device(s), such as a centrifuge or rotary filter, where the PTA solids are separated from the purification mother liquor stream, washed and then dried. The PTA product is suitable for the manufacture of polyester polymer for fibers, bottles, containers and other molded products.

The oxidation reaction is maintained at a constant temperature by evaporation of the oxidation solvent which exits the reactor and returning condensed solvent, which can also be further cooled, to the reactor. In this way, the latent heat of the oxidation solvent is used to cool the oxidation reaction mixture. The vapor phase leaving the reactor, as vent gas, typically comprises vaporized acetic acid, water vapor and volatile reaction by-products, as well as non-condensable components including residual oxygen not consumed in the oxidation reaction, nitrogen (when air is used as a source of the molecular oxygen for the oxidation reaction) and carbon oxides.

Typically, water in the oxidation solvent in the oxidation reactor is maintained at a constant level by condensing the off-gas from the oxidation reactor to form a condensate, separating the condensate from the remaining gas stream and separating at least a portion of the water from the rest of the liquid condensate, before returning the remaining liquid condensate to the reactor as oxidation solvent. The excess water separated from the condensate can be fed to an effluent treatment unit for disposal.

The separation of water from the oxidation reactor off-gas condensate can be carried out by distillation, with the lower aliphatic monocarboxylic acid-rich stream as the bottoms product and a water-rich stream as the tops product. A previous improvement to the production process was to eliminate the initial condensation step and consisted of feeding the oxidation reactor off-gas directly to a rectifier column. This column can be conveniently located above the oxidation reactor for the lower aliphatic monocarboxylic acid-rich stream to return directly to the oxidation reactor, although other configurations can also be used.

Using distillation to separate the lower aliphatic carboxylic acid from water recovered as condensate from the oxidation reactor vent gas requires a large number of distillation stages in the rectification column and sufficient aqueous reflux to the top of the column. However, the total flow of aqueous reflux to the top of the column is constrained by maintaining the oxidation reactor water concentration at a low value, to maintain the reactivity in the oxidation reactor. Typically, the aqueous reflux comprises a portion of the overheads product after condensing the water-rich vapor stream leaving the top of the rectifier column. The rest of the rectification column condensate, including water of reaction, is then removed from the rectifier overheads system.

The oxidation reactor operates at elevated pressure and temperature and the vent gas from the oxidation reactor can be used to recover energy downstream of the rectification column. Energy recovery can be either direct or indirect; by heat exchange, for example to raise steam for use elsewhere in the process or by reducing the pressure of the gas stream through a machine, such as an expander. The expander can be used to recover energy, e.g. to power the air compressor feeding air to the oxidation process or to generate electricity.

Methyl acetate is a by-product of the oxidation reaction and a volatile component that needs to be separated from the rectifier overheads vapor stream, recovered and fed back to the oxidation reaction to avoid losses from the oxidation stage. However, hydrolysis of methyl acetate to methanol and acetic acid occurs at the range of temperatures and pressures typically used in the rectification column and can reduce the efficiency of the separation of aliphatic carboxylic acid and water in the oxidation reactor vent gas and increase losses of solvent from the manufacturing process.

An existing and alternative process to reduce acetic acid losses and energy consumption during the manufacture of PTA is to extract acetic acid from waste water streams using liquid extraction and to feed the extracted stream to a downstream dehydration distillation column. Also, extraction can be used downstream of a water-acetic acid distillation column operating at atmospheric pressure. Both of these options are less energy efficient than operating at higher process pressures. Additionally, certain extraction techniques have detrimental effects on the operation of the oxidation reactor and to the CTA product produced; for example, using high boiling point solvents for high boiling point liquid extraction.

### SUMMARY OF THE INVENTION

The consequence of these combined problems reduces the economic benefits of using a rectification column to separate water from the oxidation solvent and preventing losses of acetic acid and methyl acetate from the manufacturing process. Additionally, a consequence of the high number of theoretical distillation stages necessary to separate the lower aliphatic carboxylic acid from water recovered as condensate from the oxidation reactor vent gas is the size of the rectifier, which will be a large vessel or multiple vessels and the high capital cost for this step in the production process. Because the PTA process uses corrosive solvents, such as acetic acid, and runs at high temperature and pressures, the rectifier must be constructed of expensive and corrosion resistant materials (e.g. titanium).

Therefore, a need exists to improve the method of operation of a rectifier as a distillation device. Specifically, there is a need to beneficially separate water and oxidation solvent from the oxidation reactor vent gas, to reduce the cost of separating water from the oxidation solvent, to improve the recovery of reaction byproducts, such as methyl acetate and the aliphatic carboxylic acid used in the oxidation reaction solvent, and to reduce the size of the rectification column, without increasing the effluent treatment load.

In one aspect, a method for producing an aromatic polycarboxylic acid is disclosed comprising: a) separating an oxidation reactor vent gas stream into an acetic acid rich stream and a water rich vapor stream, wherein the water rich vapor stream comprises volatile compounds and non-condensable gases, and said separating is performed in a distillation device; b) condensing said water rich vapor stream into a condensate stream and a vapor stream; c) feeding a first portion of said condensate stream to said distillation device and feeding a second portion of said condensate stream to a water treatment column to form a water treatment column bottoms stream substantially free of volatile compounds; d) feeding a portion of said water treatment column bottoms stream to a solvent recovery process comprising an extraction column; and e) separating said water treatment column bottoms stream into an acetic acid rich stream and a water rich stream. The distillation device can be a rectifier. Additionally, the acetic acid rich stream can be fed to an extraction column to separate out the extractant from the acetic acid. Similarly, the water rich stream can be fed to an extraction column to separate out the extractant. All or a portion of the acetic acid stream, either before or after extractant removal, can be fed to the oxidation reactor.

In another aspect, a method of reducing methyl acetate hydrolysis in the distillation of aromatic polycarboxylic acid oxidation vent gas is disclosed comprising: a) separating an oxidation reactor vent gas stream into an acetic acid rich stream and a water rich vapor stream, wherein the water rich vapor stream comprises volatile compounds and non-condensable gases, and said separating is performed in a distillation device; and b) condensing said water rich vapor stream into a condensate stream and a vapor stream; wherein said methyl acetate hydrolysis is reduced by about 30% to about 70% w/w and wherein the water rich vapor stream from said distillation device comprises less than about 12% w/w acetic acid. The distillation device can be a rectifier. The reduction in methyl acetate hydrolysis occurs by reducing the liquid residence time in the distillation device, when compared to the residence time in a distillation device with no subsequent solvent extraction,

In a further aspect, a method for producing terephthalic acid is disclosed comprising: a) adding paraxylene, molecular oxygen, and acetic acid to an agitated oxidation reactor; b) removing reactor vent gas from said oxidation reactor, wherein said reactor vent gas comprises acetic acid and water vapor; c) feeding said reactor vent gas to a distillation column, wherein the reactor vent gas is separated into i) an acetic acid rich stream that is fed back to said oxidation reactor, and ii) a water vapor rich stream that is fed to a condenser; d) condensing said water vapor rich stream into a condensate stream and a vapor stream, wherein a first portion of said condensate stream is fed back to said distillation column and a second portion of said condensate stream is fed to a water treatment column to form a water treatment column bottoms stream; e) feeding a portion of the said water treatment column bottoms stream to a solvent recovery process, comprising an extraction column; f) feeding said vapor stream to an absorber to remove volatile components retained in the vapor; g) feeding an extractant to said extraction column in a direction that is countercurrent to said water treatment column bottoms stream; and h) extracting acetic acid from said water treatment column bottoms stream to form an aqueous stream and an organic stream comprising acetic acid. The distillation column can be a rectifier. The organic stream comprising acetic acid can be fed directly to the oxidation reactor. Alternatively, the organic stream can be fed to an extractant stripping column to separate the extractant from the organic stream and recover the organic stream, including acetic acid, prior to feeding the stream to the oxidation reactor. Additionally, the aqueous product stream can be fed to an extractant stripping column to separate the extractant from the aqueous stream and recover the aqueous stream.

### BRIEF DESCRIPTION OF THE DRAWINGS

Figure 1 is a schematic process diagram of one aspect of the disclosed process, which illustrates a continuous oxidation process showing the configuration of the rectifier, the water treatment column and the extraction stage.
Figure 2 is a schematic process diagram of another aspect of the disclosed process, illustrating a continuous extraction process, which can be used as the extraction stage of the disclosed process.

### DETAILED DESCRIPTION

Unless defined otherwise, all technical and scientific terms used herein have the same meaning as commonly understood by one of ordinary skill in the art to which this disclosure belongs. Any methods and materials similar or equivalent to those described herein can also be used in the practice or testing of the present disclosure.

Disclosed is an improved method that increases the recovery of process solvent in the oxidation stage of the process for the manufacture of aromatic carboxylic acids. Specifically, the process combines a reduced separation of water and acetic acid in a distillation device with low-boiling point solvent extraction of the rectifier overheads condensate. The reduced separation in the distillation device lowers the duty on the device and allows for a lower number of separation stages and thus reduced height and materials cost. The solvent recovery process separates the remaining acetic acid and other organic compounds from an aqueous phase that is mostly water. This can be done in two steps: (1) a water treatment step, whereby organic compounds, such as p-xylene, are removed from an aqueous phase; and (2) an extraction step whereby the aqueous phase from the bottoms of the water treatment step is subject to solvent extraction to separate out the acetic acid and additional organic compounds into an acetic acid / organic stream. Both the acetic acid / organic stream and the aqueous phase stream can be subject to further separation steps to remove the extractant. The acetic acid / organic stream, either before or after additional extractant separation, can be fed to the oxidation reactor. Additionally, the organic compounds from the water treatment step can be fed to the oxidation reactor. The acetic acid stream can contain additional organic compounds, such as methyl acetate, methanol, and aromatic reaction intermediates. The overall process improves the acetic acid recovery for the PTA manufacturing process, while reducing the hydrolysis of methyl acetate and also reducing the effluent load on the effluent treatment plant. Losses of methyl acetate by hydrolysis are reduced by, in the range from about 30% to about 70%, compared to a PTA manufacturing process using a rectifier with no solvent extraction by reducing the liquid holdup or residence time in the distillation device as a result of reducing the acetic acid-water separation duty in the distillation device. The liquid holdup is reduced from about 20% to about 80% compared to the holdup in a distillation device with no solvent extraction, including from about 20% to about 70%, from about 20% to about 60%, from about 20% to about 50%, and from about 20% to about 40%.

Alternative separation techniques, such as membrane separation and adsorption can be used in conjunction with a rectifier to reduce the number of theoretical stages required for this duty. Required for membrane separation are high pressures, large membrane surface areas, low temperatures, and high recycle flows. Adsorption requires multiple streams and comparatively large vessels together with batch operation to regenerate the adsorption beds.

Suitable solvents such as aliphatic esters, including methyl acetate, ethyl acetate, isopropyl acetate, n-propyl acetate, methylpropyl ketone, methyl isobutyl ketone and methyl-tert-butyl ether and combinations thereof can be used as low-boiling point extractants.

The production of aromatic carboxylic acids, including terephthalic acid, can take place in an agitated oxidation reactor. Here, an aromatic feedstock, e.g. paraxylene, is reacted with molecular oxygen, typically derived from air, in the presence of a reaction catalyst and aqueous acetic acid solvent, to produce the carboxylic acid. The reaction temperature can be between about 150°C to about 250°C, including 190°C; and the pressure can be between about 6 bar absolute (barA) to about 30 barA, including 13 barA. CTA solids are precipitated in the reactor as the product of the oxidation reaction and maintained in suspension by an agitator. Other feed streams to the oxidation reactor can comprise reflux solvent, recycled solvent, recovered paraxylene and recovered methyl acetate. Also, the oxidation reaction intermediates can be recovered from the pure plant mother liquor and recycled to the oxidation reactor. This increases the efficiency and conversion of feedstock to aromatic carboxylic acid product.

The slurry of CTA in the oxidation solvent (mother liquor) flows to crystallizers downstream of the oxidation reactor. The CTA solids are then separated from the oxidation mother liquor using a rotary filter, a centrifuge, or other similar devices. The separation temperature ranges from about 90°C to about 160°C, and the pressure from about 0.5 barA to about 4.5 barA.

The oxidation reaction is exothermic and the heat of reaction is removed by evaporation of solvent into the reactor vent gas that flows to a distillation device, such as a rectifier, which can be one or more vessels. Acetic acid and water in the reactor vent gas are separated by distillation, which operates with the distillation column overheads temperature in the range from about 140°C - 200°C, including about 170°C.

Aqueous reflux is supplied to the top of the distillation column from the overheads condensers, which can comprise one or more heat exchangers. Additional aqueous reflux comprising pure plant mother liquor can be fed at or below the top of the column. An acetic acid rich stream from the base of the column can be returned to the oxidation reactor. The base of the column operates at about the same temperature as the oxidation reactor.

The water-rich vapor from the top of the column comprises acetic acid, typically about 0.1 to about 12% w/w, including from about 0.1 to about 5% w/w, about 3 to about 5% w/w, and about 5% w/w, which is condensed and a portion of the condensate, typically at a temperature in the range from about 130°C to about 160°C, is fed to the top of the column as aqueous reflux. The rest of the condensate is cooled down in stages to a temperature in the range from about ambient (25°C) to about 100°C, including about 40°C. Pure plant mother liquor, comprising oxidation intermediates, such as pTol, can be fed at or below the top of the distillation column, as an additional aqueous liquid feedstream at a temperature in the range of about 140°C - 160°C, including about 149°C. The overheads condensers can comprise two or more heat exchangers, typically with at least one used to generate steam for efficient heat recovery from the column overheads.

Uncondensed gas passes to an absorber at about 6 to about 30 barA to remove volatile components, such as paraxylene, methanol and methyl acetate retained in the vapor. The volatile components can be removed by contacting with liquid, first with an acetic acid rich stream, such as the oxidation solvent, and then with a water rich stream. The scrubbing liquors are fed to the oxidation reactor. Scrubbed vent gas from the top of the absorber in the range from about 4 to about 28 barA, including about 11 barA, can be processed further, including separation into a residual vent gas stream, wherein energy is recovered from the residual vent gas stream, before being vented to the atmosphere. Energy can be recovered by a mechanical device. The mechanical device can be an expander. The energy recovered can be used to general electricity.

To recover organic components in the distillation column overheads condensate, at least part of the overheads condensate from the distillation column can be fed to the water treatment column, which operates close to atmospheric pressure and at least part of the condensate stream flashes as it enters the water treatment column. Volatile components comprising methyl acetate and p-xylene are substantially separated from the aqueous stream and can be returned to the oxidation reactor. To enhance separation of the volatile components from the aqueous phase in the water treatment column, steam can be fed to the bottom of the column.

A first portion of the water treatment column bottoms stream at about 105°C can be fed to a solvent recovery process, comprising an extraction column to separate organic compounds, including acetic acid, from the water-rich water treatment column bottoms stream and a second portion can be fed to the purification section.

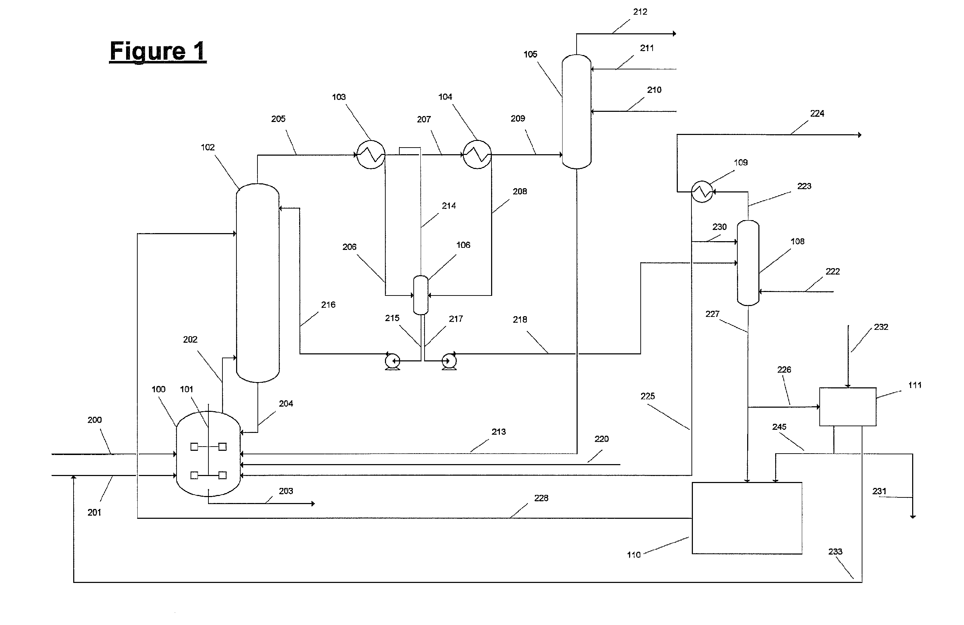

Figure 1 describes one aspect of the disclosed process using a rectifier as the distillation device / distillation column. Here, paraxylene is oxidized to CTA using molecular oxygen in an agitated reactor comprising one or more stirred vessels under elevated temperature and pressure. Specifically, the oxidation reactor 100 can be fed with air 200, aqueous acetic acid solvent 201, containing the reaction catalyst, and paraxylene 220. CTA solids are precipitated in the reactor, as the product of the oxidation reaction, and are maintained in suspension by an agitator 101. Other feed streams to the oxidation reactor can include reflux solvent 204 from rectifier 102; recycle solvent 213 from absorber 105; and recovered methyl acetate and methanol 225 from the water treatment column 108. The oxidation reaction is exothermic and the heat of reaction is removed by evaporation of solvent into the reactor vent gas 202. The reactor product 203, a slurry of CTA in the oxidation solvent, flows to more than one crystalliser in series downstream of the oxidation reactor, before the CTA solids are separated from the oxidation mother liquor using a suitable device.

The reactor vent gas 202 from the oxidation reactor 100 flows to a rectifier 102. Acetic acid and water in the reactor vent gas are separated by distillation in the rectifier. Aqueous reflux is supplied to the top of the rectifier via stream 216, which is a portion of the condensate produced in the overheads condensers 103 and 104, comprising one or more heat exchangers. Additional aqueous reflux 228 can be fed at or below the top of the rectifier, comprising pure plant mother liquor, resulting from the separation of PTA solids following crystallization in the purification stage. An acetic acid rich stream 204, from the base of the rectifier can be returned to the oxidation reactor.

The vapor stream 205, containing acetic acid, flows from the top of the rectifier to the rectifier overheads condensers 103 and 104. The condensers 103 and 104 comprise two or more heat exchangers with at least one used to generate steam for efficient heat recovery. The vapor 205 from the top of the rectifier is condensed and cooled down in stages. The condensate 206 and 208 can be separated from the vapor stream at each stage of condensation and flowed to a reflux pot 106, which can be pressure balanced, for example, via line 214 to the overheads vapor line 207. A portion of the condensate collected in reflux pot 106 flows to the top of the rectifier as aqueous reflux 215 and 216. Uncondensed gas 209 from the final heat exchanger passes to an absorber 105 to remove volatile components retained in the vapor. The volatile components are removed by contacting with liquids, including, first with an acetic acid rich stream, such as the oxidation solvent 210, and then with a water-rich stream 211. The scrubbing liquors 213, comprising acetic acid and water from the bottom of the absorber, flow to the oxidation reactor 100. Scrubbed vent gas 212 from the top of the absorber passes forward for further processing, including energy recovery, before being vented to the atmosphere.

Condensate 217 and 218 collected in the reflux pot 106 can also flow to the water treatment column 108, where volatile components are separated from the aqueous product and recovered in the top vapor 223 which flows to condenser 109 where it is substantially condensed. A portion of the condensate 230 from the water treatment column overheads condenser 109 is returned as reflux to the column, while the rest 225 can be returned to the oxidation reactor 100. Non-condensable vapors 224 from the water treatment column overheads condenser 109 can be vented from the system.

To ensure separation of the volatile components from the aqueous phase, steam 222 can be fed to the bottom of the water treatment column 108. Water from the base of the water treatment column 227 can be used elsewhere in the production process, including the purification plant 110. A portion of the water from the base of the water treatment column 226 is fed to the solvent recovery system 111, where residual organic compounds, comprising acetic acid are recovered and fed 233 to the oxidation reactor and at least a portion of the extracted aqueous stream from the solvent recovery system is fed 245 to the purification process 110. Excess water 231 can be fed to other process users or to effluent. Extractant is fed 232 to the solvent recovery system 111, as required, e.g. to make up for losses.

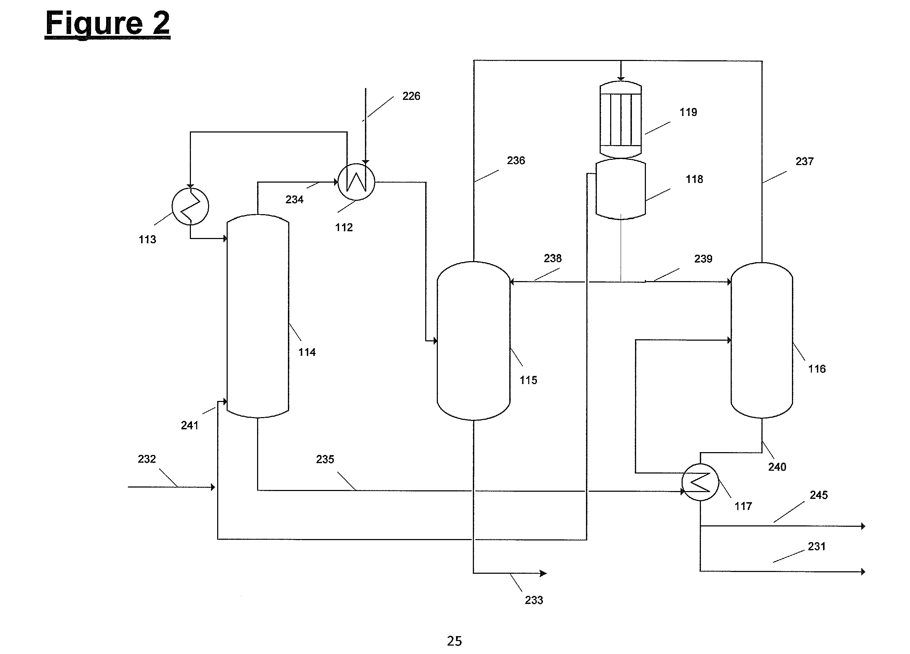

The solvent recovery system 111 is described in Figure 2. The condensate from the rectifier overheads condensers collected in reflux pot 106 comprises acetic acid that can be recovered by extraction to reduce the amount of oxidation process solvent fed to effluent treatment and to the purification process and reduce losses in the rest of the PTA production process.

In one aspect of the disclosed process, a liquid aqueous stream 226 comprising less than about 20 %w/w acetic acid is cooled in an interchanger 112 and a trim cooler 113 to about 40°C before feeding to the extraction column 114, where the aqueous stream is fed to the top of the column and flows down, counter-current to the organic phase, fed to the bottom of the extraction column. The aqueous phase product 235 exits the bottom of the extraction column and is heated in an interchanger 117 before feeding the extractant stripping column 116, to remove dissolved organic extractant. The water-rich product stream 240 from the base of the extractant stripping column is cooled from about 109°C to about 50°C in interchanger 117 before being used elsewhere in the manufacturing process or purged to effluent (245 and 231). The extractant-rich overheads vapor 237 exits the top of the extractant stripping column at a temperature in the range about 71°C to about 100°C, including about 93°C and is condensed in the overheads condenser 119 and flows to a decanter 118. Separation of the organic and aqueous phases takes place in the decanter, with the aqueous phase as reflux 238, 239 flowing to the extractant rectification column 115 and extractant stripping column 116. The organic phase 241 flows to the bottom of the extraction column 114, flows up the column counter-current to the aqueous feed stream, exits the top of the extraction column 234 and is heated in interchanger 112, before feeding the extractant rectifier column 115. Extractant-rich overheads vapor 236 exits the top of the extractant rectifier column at a temperature in the range from about 71°C to about 100°C, including about 79°C and is condensed in the overheads condenser 119 and flows to a decanter 118. The acetic-rich solvent organic stream exits the base of the extractant rectifier column 115 at about 119°C and is recycled 233 to the oxidation reactor 100. The extractant can be an aliphatic ester, including a C1-C3 alkyl ester, such as ethyl acetate, or a C4-C5 alkyl ketone.

### EXAMPLES

The following examples further illustrate the various aspects of the disclosed processes.

A combination of physical measurements and modeling give the results in the examples.

### Example 1

A rectifier is configured, as shown in Figure 1, to receive the vent gas from a CTA oxidation reactor and reflux an acetic acid-rich stream back to the reactor. The overheads from the rectifier are substantially condensed and a portion of the condensate is returned to the top of the rectifier as liquid reflux and the remainder is fed to the water treatment column. The overhead vapor from the water treatment column is substantially condensed and a portion of the condensate is returned to the water treatment column as reflux and the remainder can be recycled to the oxidation reactor. Water from the base of the water treatment column is fed to a solvent recovery system, where acetic acid is substantially separated from the aqueous stream and recovered to the process.

Table 1 shows the concentration of acetic acid at key locations in the PTA production process and methyl acetate hydrolysis data for various aspects of the disclosed process as well as a comparative embodiment without a solvent recovery system.

### Example 2

A system is configured as for Example 1, but the rectifier has a lower water - acetic acid separation capacity by reducing the number of theoretical stages.

As shown in Table 1, the disclosed process can result in a higher concentration of acetic acid in the rectifier overheads vapor, resulting in a higher load for the solvent recovery system. The reduced size of the rectifier and a modest increase in the size of the solvent recovery system enable the disclosed process to efficiently produce pure PTA at a lower cost without further losses of oxidation process solvent.

### Comparative example 1

A system is configured as for Example 1, but without a solvent recovery system comprising an extraction column to remove acetic acid from a portion of the water stream from the base of the water treatment column.

**Table 1**

| Example | **Example 1** | **Example 2** | **Comparative Example 1** |
|---|---|---|---|
| Acetic acid concn. in Rectifier overheads vapor % w/w | 0.9 | 6 | 0.9 |
| Acetic acid concn. in stream to solvent recovery system % w/w | 1.5 | 10 | NA |
| Acetic acid recovered by solvent recovery system % | 93 | 99 | NA |
| Acetic acid concn. in aqueous effluent % w/w | 0.1 | 0.1 | 1.5 |
| Relative aqueous effluent load | 1 | 1 | 15 |
| Theoretical stages in rectifier | 46 | 16 | 46 |
| Methyl acetate concn. in Rectifier overheads vapor % w/w | 0.95 | 0.96 | 0.95 |
| Total Methyl acetate hydrolysis in Rectifier (kmol/hr) | 14.9 | 10.0 - 4.9* | 14.9 |
| Relative Methyl acetat e hydrolysis | 1 | 0.7 - 0.3* | 1 |

| | | | |
|---|---|---|---|
| * Dependant on liquid hold-up in column | | | |

While the invention has been described in conjunction with specific embodiments thereof, it is evident that many alternatives, modifications and variations will be apparent to those skilled in the art in light of the foregoing description. Accordingly, the invention is intended to embrace all such alternatives, modifications and variations as fall within the spirit and scope of the claims.

### FURTHER EMBODIMENTS OF THE INVENTION

1. An aromatic polycarboxylic acid manufacturing process comprising:
   a) separating an oxidation reactor vent gas stream into an acetic acid rich stream and a water rich vapor stream, wherein the water rich vapor stream comprises acetic acid, volatile compounds and non-condensable gases, and said separating is performed in a distillation device;
   b) condensing said water rich vapor stream into a condensate stream and a vapor stream;
   c) feeding a first portion of said condensate stream to said distillation device and feeding a second portion of said condensate stream to a water treatment column to form a water treatment column bottoms stream substantially free of volatile compounds;
   d) feeding a portion of the said water treatment column bottoms stream to a solvent recovery process comprising an extraction column; and
   e) separating said water treatment column bottoms stream into an acetic rich stream and a water rich stream.
2. The process of embodiment 1, wherein said distillation device comprises a rectifier.
3. The process of embodiment 1 further comprising feeding a portion of the acetic acid rich stream from the said solvent recovery process to said oxidation reactor.
4. The process of embodiment 1 wherein liquid-liquid extraction is used to separate acetic acid from said water treatment column bottoms stream.
5. The process of embodiment 4 wherein the liquid extractant is a C1-C3 alkyl ester or C4-C5 alkyl ketone.
6. The process of embodiment 4 further comprising d) separating the liquid extractant from said extracted water treatment column bottoms stream to generate a water stream that is substantially free of acetic acid.
7. The process of embodiment 6, wherein the resultant water stream is used in the manufacture of aromatic carboxylic acids.
8. The process of embodiment 7, wherein said aromatic carboxylic acid is terephthalic acid.
9. The process of embodiment 8 wherein the liquid extractant is ethyl acetate.
10. The process of embodiment 8 wherein the height of the distillation device is reduced by decreasing the number of separation stages and reducing the separation duty of the distillation device.
11. The process of embodiment 1 further comprising separating the oxidation reactor vent gas stream into a residual vent gas stream, wherein energy is recovered from the residual vent gas stream.
12. The process of embodiment 11, wherein said energy recovery is by a mechanical device.
13. The process of embodiment 12, wherein said mechanical device is an expander.
14. The process of one of embodiments 11-13, wherein the recovered energy can be used to generate electricity.

## Claims

1. A method of reducing methyl acetate hydrolysis in the distillation of aromatic polycarboxylic acid oxidation vent gas comprising:
a) separating an oxidation reactor vent gas stream into an acetic acid rich stream and a water rich vapor stream, wherein the water rich vapor stream comprises volatile compounds and non-condensable gases, and said separating is performed in a distillation device; and
b) condensing said water rich vapor stream into a condensate stream and a vapor stream; wherein said methyl acetate hydrolysis is reduced by about 30% to about 70% w/w and wherein the water rich vapor stream from said distillation device comprises less than about 12% w/w acetic acid.

2. The method of claim 1, wherein said methyl acetate reduction is achieved by reducing the liquid residence time in the distillation device, when compared to the residence time in a distillation device with no solvent extraction.

3. A terephthalic acid manufacturing process comprising:
a) adding paraxylene, molecular oxygen, and acetic acid to an agitated oxidation reactor;
b) removing reactor vent gas from said oxidation reactor, wherein said reactor vent gas comprises acetic acid and water vapor;
c) feeding said reactor vent gas to a distillation column, wherein the reactor vent gas is separated into an acetic acid rich stream that is fed back to said oxidation reactor and into a water vapor rich stream that is fed to a condenser;
d) condensing said water vapor rich stream into a condensate stream and a vapor stream, wherein a first portion of said condensate stream is fed back to said distillation column and a second portion of said condensate stream is fed to a water treatment column to form a water treatment column bottoms stream;
e) feeding a portion of the said water treatment column bottoms stream to a solvent recovery process, comprising an extraction column;
f) feeding said vapor stream to an absorber to remove volatile components retained in the vapor;
g) feeding an extractant to said extraction column in a direction that is countercurrent to said water treatment column bottoms stream; and
h) extracting acetic acid from said water treatment column bottoms stream to form an aqueous stream and an organic stream comprising acetic acid.

4. The process of claim 3, wherein said distillation column is a rectifier.

5. The process of claim 3, wherein said extractant is ethyl acetate.

6. The process of claim 3, further comprising feeding said organic stream to the oxidation reactor.

7. The process of claim 3, further comprising:
i) feeding said aqueous product stream to an extractant stripping column to separate the extractant from the aqueous stream and recover the aqueous stream;
j) feeding the organic stream to an extractant stripping column to separate the extractant from the organic stream to create an extractant free organic stream; and
k) feeding the extractant free organic stream to the oxidation reactor.
